# EUROPÄISCHE PATENTSCHRIFT

(11) **EP 0 703 795 B1**
(45) Veröffentlichungstag und Bekanntmachung des Hinweises auf die Patenterteilung: **28.03.2001**
(21) Anmeldenummer: 94918271.1
(22) Anmeldetag: 15.06.1994
(51) Int. Cl.: A61M 3/02, A61B 17/34

(54) **IN DEN MENSCHLICHEN KÖRPER EINFÜHRBARES INSTRUMENT**
INSTRUMENT CAPABLE OF BEING INTRODUCED INTO THE HUMAN BODY
INSTRUMENT INTRODUISIBLE DANS LE CORPS HUMAIN

(30) Priorität: 15.06.1993 DE 4319630
(43) Veröffentlichungstag der Anmeldung: 03.04.1996
(73) Patentinhaber: Storz Endoskop GmbH, 8200 Schaffhausen (CH)
(72) Erfinder: NOVAK, Pavel, CH-8200 Schaffhausen (CH); KRAFT-KIVIKOSKI, Jürgen, D-78315 Radolfzell (DE); WEHRSTEIN, Helmut, D-78532 Tuttlingen (DE)
(74) Vertreter: Münich, Wilhelm, Dr.
(86) Internationale Anmeldenummer: DE9400663
(87) Internationale Veröffentlichungsnummer: WO9428952

(56) Entgegenhaltungen:
- EP-A- 0 201 984
- EP-A- 0 564 953
- WO-A-91/17112
- WO-A-92/12694
- WO-A-92/12759
- US-A- 4 248 217
- US-A- 4 682 010
- Prospekt Medizintechnik GmbH: Hypothermie Prävention
- Prospekt Hoyer Bremen: Die Wärme des Lebens; Level 1
- Propekt Hoyer Bremen: Level 1, Normothermie-System

## Beschreibung

### Technisches Gebiet

Die Erfindung bezieht sich auf ein endoskopisches Instrument gemäß dem Oberbegriff des Patentanspruchs 1.

Beispielsweise bei laparoskopischen Eingriffen ist es in der Regel erforderlich ein Fluid und insbesondere ein Gas, wie beispielsweise CO2, in die Körperhöhle einzuleiten, in die der Einstich erfolgt ist, damit die Körperhöhle nicht durch den "Außendruck" zusammengedrückt wird.

Mit zunehmender Operationsdauer, der Anzahl der Einstiche sowie der Häufigkeit des Instrumentenwechsels werden nicht nur relativ große Mengen an Insufflations-Gas, sondern auch hohe Insufflationsraten benötigt. Dies trifft erst recht auf die HF-Chirurgie und Laserbehandlungen zu, bei denen das Gas zusätzlich auftretenden Rauch etc. abführen muß. Während in der Vergangenheit Flußraten von wenigen Litern pro Minute ausreichend gewesen sind, werden seit einiger Zeit immer höhere Flußraten verlangt.

Bereits bei Flußraten im Bereich von 5 bis 7 l/min kann jedoch eine Unterkühlung des Patienten, d.h. eine lokale Absenkung der Temperatur deutlich unter die Körpertemperatur auftreten.

Werden (Insufflations)-Geräte mit noch höheren Leistungen, die bei auf dem Markt befindlichen Geräten bis zu 15 l/min reichen, oder gar Insufflationsgeräte mit Durchflußraten bis zu 40 l/min verwendet, wie sie in der Patentliteratur beschrieben sind, so wird das Problem der Unterkühlung noch viel gravierender als bei herkömmlichen Geräten.

### Stand der Technik

Es ist deshalb vorgeschlagen worden, in dem Insufflationsgerät eine Heizung für das Fluid vorzusehen. Insbesondere bei Gas-Insufflationsgeräten tritt bei der Aufheizung des Gases im Insufflationsgerät das Problem auf, daß aufgrund der geringen Wärmekapazität des Gases dieses auf dem Weg zum Patienten wieder im Zuleitungsschlauch abgekühlt wird.

Deshalb ist es immer dann, wenn die Erwärmung des Gases nur im Insufflationsgerät erfolgt, erforderlich, das Gas auf eine höhere Temperatur als eigentlich gewünscht aufzuheizen, damit das Gas beim Eintritt in den menschlichen Körper "gerade" eine Temperatur entsprechend der Körpertemperatur (37°C) hat. Dies hat jedoch den Nachteil, daß aufgrund des normalerweise erforderlichen langen Schlauches die Trägheit des Systems bei Abweichungen der Temperatur von der Soll-Temperatur, d.h. die Regelkonstante groß und damit aufgrund von Regelschwankungen die Temperaturkonstanz gering ist, so daß unter Umständen sogar das Gas mit zu hoher Temperatur in das Körperinnere eingeleitet wird.

Bei einem weiteren Lösungsversuch des Problems der Unterkühlung des Patienten, nämlich einem u.a. unter der Bezeichnung "Flow-Therme" vertriebenen Gerät der Fa. Wisap, Sauerlach, wird zusätzlich ein heizbarer Schlauch verwendet, der das eigentliche Insufflationsgerät mit dem in den menschlichen Körper einführbaren Instrument verbindet.

Hierzu wird auch auf die EP-A-0 564 953 verwiesen, die nach dem Prioritätsdatum der vorliegenden Anmeldung veröffentlicht worden ist, und in der ein endoskopisches Instrument beschrieben ist, von dem bei der Formulierung des Oberbegriffs des Patentanspruchs 1 ausgegangen wird.

Auch dieser Lösungsversuch gewährleistet keine optimale Temperaturregelung, da die Regelkonstante aufgrund der Schlauchlänge und der längs des Schlauches unter Umständen stark variierenden Umgebungsbedingungen groß ist. Darüberhinaus ist ein heizbarer Schlauch aufgrund der integrierten Heizung weniger biegsam sowie schwerer als ein normaler Schlauch, so daß er nicht so leicht handhabbar wie ein normaler Schlauch ist.

### Darstellung der Erfindung

Der Erfindung liegt die Aufgabe zugrunde, bei der Einleitung wenigstens eines Gases in das Körperinnere eine hervorragende Temperaturregelung und insbesondere Temperaturkonstanz des in den Körper eingeleiteten Gases sicherzustellen, ohne daß die Bewegungsfreiheit des Operateurs behindert wird .

Eine erfindungsgemäße Lösung dieser Aufgabe ist im Anspruch 1 angegeben. Weiterbildungen der Erfindung sind Gegenstand der Unteransprüche.

Erfindungsgemäß weist das Instrument eine Heizeinrichtung für das Gas auf, die an bzw. in einem weiteren Kanal vorgesehen ist. Diese Heizeinrichtung kann das Gas alleine oder zusätzlich zu einer Heizeinrichtung im Insufflationsgerät sowie gegebenenfalls einer "unterstützenden" Heizung im Insufflationsschlauch aufheizen.

Bevorzugt ist in jedem Falle, daß die Heizeinrichtung des Instruments die "Führung" des Regelvorgangs "übernimmt".

Dadurch, daß die Heizeinrichtung im bzw. unmittelbar vor dem menschlichen Körper, nämlich in dem oder bei dem in den menschlichen Körper eingesetzten Instrument angeordnet ist, wird sichergestellt, daß eine optimale Temperaturregelung mit kurzen Regelkonstanten erfolgen kann, so daß das Gas mit hervorragender Temperaturkonstanz in den Körperhohlraum eingeleitet wird.

Ein weiterer Vorteil des erfindungsgemäßen Instruments liegt darin, daß auch das Instrument sowohl durch die Heizung selbst als auch durch das erwärmte Fluid mit beheizt wird. Hierdurch wird ein Beschlagen der Instrumente und insbesondere einer im Instrumente angeordneten Endoskopoptik vermieden. Dabei ist es von besonderem Vorteil, wenn die Heizeinrichtung den Teil des Instruments umgibt, der das optische System des Endoskops aufnimmt, da hierdurch eine optimale Beheizung nicht nur des Fluids, sondern auch der "sensiblen" Teile des Instruments gewährleistet ist.

Weiterhin kann ohne Beeinträchtigung der Temperatur-Regelqualität ein konventioneller Zuleitungsschlauch mit beliebiger und insbesondere von Eingriff zu Eingriff variabler Länge zwischen Insufflationsgerät und Instrument eingesetzt werden.

Weiterbildungen der Erfindung sind in den Ansprüchen 2 bis 17 angegeben.

Gemäß Anspruch 2 kann die Heizeinrichtung in das Instrument selbst integriert sein. Diese Ausbildung hat nicht nur den Vorteil, daß gegebenenfalls die in dem Instrument befindliche Optik direkt mitgeheizt wird, sondern auch den - auf den ersten Blick nicht sofort erkennbaren -Vorteil, daß die Gewichtsbalance des Instruments verbessert wird:

Obwohl man in der Regel bemüht ist, die Instrumente so leicht wie möglich zu gestalten, wird diese Regel bei Laparoskopen, die in Verbindung mit einem C02-Laser eingesetzt werden, durchbrochen. Um das Gewicht des C02-Lasers bzw. des mit diesem Laser verbundenen Gelenkarms zu kompensieren, ist es erforderlich, das Instrument schwerer als üblich zu gestalten. Dieser Forderung kann durch den Einbau der Heizeinrichtung in das Instrument ohne weiteres entsprochen werden.

Alternativ kann die Heizeinrichtung in einem separaten Gehäuse angeordnet sein, das einen Fluideinlaß und einen Fluidauslaß aufweist, der über ein kurzes Schlauchstück mit dem proximalen Fluideinlaß des eigentlichen Instruments verbunden ist. Durch diese Ausbildung wird nicht nur die in bestimmten Anwendungsfällen unerwünschte Kopflastigkeit vermieden, sondern es ist auch möglich, bereits vorhandene Instrumente leicht so nachzurüsten, daß sie dem erfindungsgemäß vorgeschlagenen Instrument funktionell entsprechen.

In den Ansprüchen 4 bis 7 ist die Verwendung einer elektrischen Heizwendel und deren spezielle Ausgestaltung gekennzeichnet.

Bei der vorstehend angegebenen Alternative, bei der die Heizeinrichtung außerhalb des menschlichen Körpers angeordnet ist, ist es bevorzugt, wenn die Heizeinrichtung in dem Teil des Instruments vorgesehen ist, der außerhalb des menschlichen Körpers verbleibt. Hierdurch ist es möglich, im dicker als gewöhnlich gestalteten proximalen Teil des Instruments die Heizeinrichtung sowie Teile der Steuereinheit unterzubringen, ohne die Funktionalität des Instruments zu beeinträchtigen.

Eine weitere Alternative, die sich insbesondere zur Nachrüstung vorhandener Instrumente eignet, ist im Anspruch 10 angegeben:

Die Heizeinrichtung ist in einem separaten Gehäuse vorgesehen, das einstückig mit dem in herkömmlicher Weise ausgebildeten Instrument verbunden ist. Diese Alternative erlaubt ebenfalls das Nachrüsten von vorhandenen Instrumenten.

Im Anspruch 11 ist angegebenen, daß die Heizeinrichtung wenigstens einen Temperaturfühler aufweist, dessen Ausgangssignal an eine Regeleinrichtung angelegt ist, die die Leistung regelt, mit der die Heizeinrichtung beaufschlagt wird. Damit ist nicht nur eine Steuerung der Heizleistung, sondern auch eine Regelung der eingestellten Temperatur möglich.

Bei der im Anspruch 12 angegebenen Weiterbildung sind mindestens zwei Temperaturfühler vorgesehen, deren Ausgangssignale zur Überprüfung der Funktion der Temperaturfühler verglichen werden. Damit ist es möglich, Fehlfunktionen verhältnismäßig genau zu erkennen.

Hierzu ist es von Vorteil, wenn der oder die Temperaturfühler möglichst nahe am Kanal angeordnet sind, durch den das Fluid strömt.

Unabhängig von der genauen Ausstattung des Temperaturfühlers ist es von Vorteil, wenn ein Temperaturfühler, der eine Sicherheitsabschaltung auslösen kann, möglichst nahe dem distalen Ende des Instruments angeordnet ist.

Zur Steuerung des erfindungsgemäßen Instruments können die verschiedensten Auswerteaalgorithmen verwendet werden. Diese Strategien können in einem Programmspeicher der Steuereinheit gespeichert und bei Bedarf abgerufen werden.

In jedem Fall können beliebige Instrumente und insbesondere ein Trokar sowie ein an sich bekanntes Laparoskop für operative Anwendungen in Verbindung mit der Erfindung eingesetzt werden.

### Kurze Beschreibung der Zeichnung

Die Erfindung wird nachstehend ohne Beschränkung des allgemeinen Erfindungsgedankens anhand von Ausführungsbeispielen unter Bezugnahme auf die Zeichnung exemplarisch beschrieben, auf die im übrigen bezüglich der Offenbarung aller im Text nicht näher erläuterten erfindungsgemäßen Einzelheiten ausdrücklich verwiesen wird. Es zeigen:
- Fig. 1: eine Seitenansicht eines ersten Ausführungsbeispiels eines erfindungsgemäßen Instruments,
- Fig. 2: einen Schnitt bei I-I in Fig. 1,
- Fig. 3: eine Abwandlung des in Fig. 1 gezeigten Ausführungsbeispiels,
- Fig. 4: eine weitere Abwandlung des in Fig. 1 gezeigten Ausführungsbeispiels, und
- Fig. 5: ein zweites Ausführungsbeispiel der Erfindung.

### Beschreibung von Ausführungsbeispielen

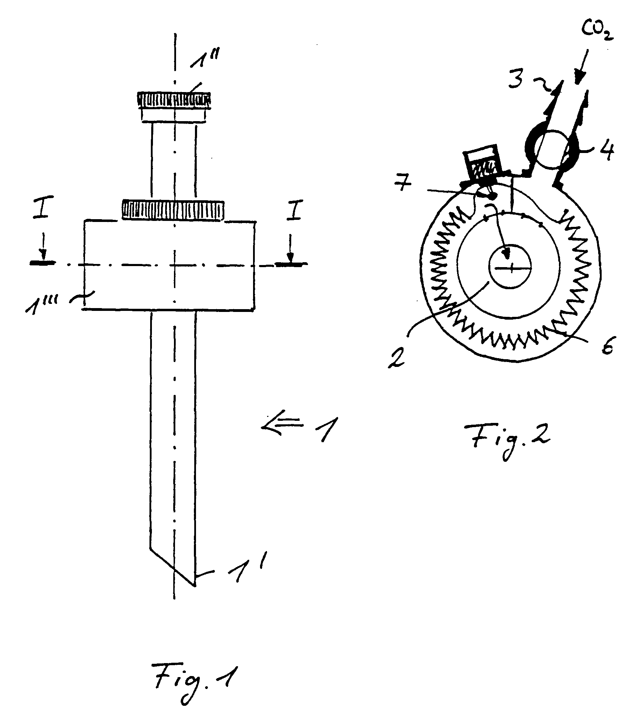

Fig. 1 zeigt ein in das Innere eines menschlichen (oder tierischen) Körpers einsetzbares Instrument, das ohne Beschränkung der Allgemeinheit ein an sich bekannter Trokar 1 ist, der ein distales Ende 1' und ein proximales Ende 1" aufweist. Der Trokar weist einen sich in Längsrichtung erstreckenden Hohlraum 2 auf, in den (beispielsweise) eine nicht dargestellte Endoskopoptik derart einsetzbar ist, daß zusätzlich ein Kanal (mit ringförmigem Querschnitt) für ein Fluid und insbesondere ein Gas, wie CO2 gebildet wird. Selbstverständlich kann aber auch ein von den übrigen einsetzbaren Instrumenten getrennter Kanal für das einzuleitende Fluid vorgesehen sein.

Für die Einleitung von Gas in den durch das Einsetzen eines Instruments gebildeten bzw. bereits vorhandenen Kanal ist ein Anschluß 3, der beispielsweise ein konventioneller Luer-Lock-Anschluß oder ein Schlauchtülle sein kann, mit einem Hahn 4 vorgesehen. Bei großen Durchflußraten kann auch ein Anschluß mit einem größeren Durchmesser als üblicherweise in Trokaren verwendet eingesetzt werden.

Für die Erwärmung des kalten oder durch eine Heizeinrichtung in einem nicht dargestellten Insufflationsgerät, das über einen Schlauch mit dem Anschluß 3 verbunden ist, bereits vorgewärmten Gases ist eine Heizeinrichtung 5 mit einem Anschluß 5' vorgesehen, die wenigstens eine elektrische Heizwendel 6 aufweist, die den Kanal, durch den das Fluid geleitet wird, umgibt.

Die Heizeinrichtung 5 ist bei dem gezeigten Ausführungsbeispiel in dem Teil des Trokars als "Verdickung" vorgesehen, der außerhalb des menschlichen Körpers verbleibt.

Weiterhin weist die Heizeinrichtung wenigstens einen Temperaturfühler 7 auf, dessen Ausgangssignal an eine nicht dargestellte Steuer- bzw. Regeleinrichtung angelegt ist, die die elektrische Leistung regelt, mit der die Heizwendel 6 beaufschlagt wird. Dabei sind bevorzugt wenigstens zwei Temperaturfühler vorgesehen, deren Ausgangssignale zur Überprüfung der Funktion der Temperaturfühler verglichen werden. Der oder die Temperaturfühler sind möglichst nahe am Kanal angeordnet, durch den das Fluid strömt, so daß die Regelkonstanten auch bei hohen Flußraten bis zu 40 l/min und mehr sowie bei stark variierenden Flußraten gering sind.

In den folgenden Figuren sind gleiche bzw. ähnliche Teile mit den selben Bezugszeichen bezeichnet, so daß auf eine erneute Vorstellung verzichtet wird.

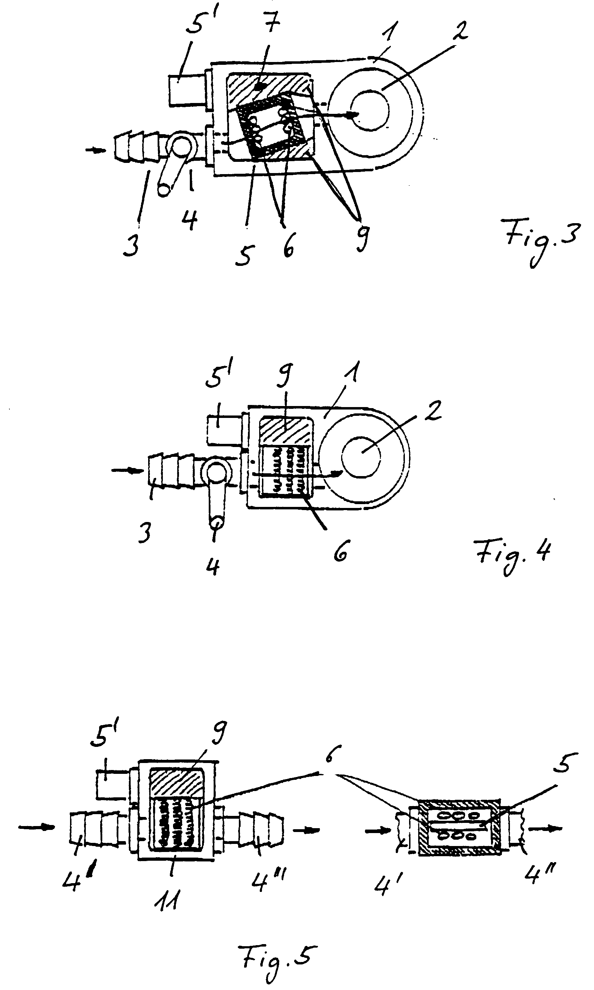

Fig. 3 zeigt eine teilweise geschnittene Aufsicht auf eine Modifikation des in den Figuren 1 und 2 gezeigten Ausführungsbeispiels. Bei diesem Ausführungsbeispiel ist die Heizeinrichtung in einem seitlichen Ansatz untergebracht. Die Heizwendel 6 ist würfelförmig ausgebildet und in dem Kanal angeordnet, der den Anschluß 3 nebst Hahn 4 mit dem Hohlraum 2 im Instrument verbindet. Mit 9 ist eine Vergußmasse bezeichnet.

Fig. 4 zeigt ebenfalls eine teilweise geschnittene Aufsicht auf eine weitere Modifikation des in den Figuren 1 und 2 gezeigten Ausführungsbeispiels. Bei dieser Modifikation ist die Heizwendel als flacher, elektrisch beheizter Heizkörper ausgebildet.

Fig. 5 zeigt ein zweites Ausführungsbeispiel der Erfindung in einer teilweise geschnittenen Aufsicht bzw. einer Seitenansicht. Bei diesem Ausführungsbeispiel ist die Heizeinrichtung nicht in dem Instrument selbst, sondern in einem Zusatzteil untergebracht, der ein Gehäuse 11 aufweist, das über eine als Einlaß fungierende Schlauchtülle 4' sowie eine als Auslaß fungierende Schlauchtülle 4" verfügt. Mittels der Schlauchtülle 4" und einem kurzen Schlauch ist das Zusatzteil mit dem Einlaß 3 des in Fig. 5 nicht dargestellten Instruments verbunden. In dem Gehäuse 11 befindet sich ein Fluidkanal, in dem wiederum die als flacher Heizkörper ausgebildete Heizwendel angeordnet ist. Mit 5' ist der Anschluß für die Heizeinrichtung bezeichnet.

Vorstehend ist die Erfindung anhand eines Ausführungsbeispiels ohne Beschränkung der Allgemeinheit beschrieben worden. Beispielsweise kann anstelle einer elektrischen Heizeinrichtung auch eine mit einem Wärmetauscher arbeitende Heizeinrichtung verwendet werden.

Selbstverständlich ist die Ausbildung der Heizwendeln nicht auf die vorstehend beschriebenen Ausführungsformen beschränkt. Vielmehr können die verschiedensten Heizkörper verwendet werden, die beispielsweise auch als "Rohr-Heizkörper" ausgeformt sein können.

Auch ist das Instrument, das erfindungsgemäß mit einer Heizeinrichtung ausgestattet ist, nicht auf die vorstehend explizit erwähnten Trokare und Laparoskope beschränkt.

## Patentansprüche

1. Endoskopisches Instrument mit
- einem außerhalb des menschlichen Körpers verbleibenden Teil, an dem ein Gas-Anschluß (3;4') vorgesehen ist, der zum Anschluß eines Verbindungsschlauches dient, der das Instrument mit einem Insufflationsgerät verbindet, das Gas, wie z.B. CO₂ mit zur Insufflation von Körperhöhlen geeigneten Flußraten, insbesondere von bis 401/min fördert, und
- einem in den menschlichen Körper einführbaren länglichen Teil, das wenigstens einen sich in Längsrichtung dieses Teils erstreckenden Kanal (2) aufweist, der über einen weiteren Kanal mit dem Gas-Anschluß (3,4') verbunden ist, so daß Gas in das Körperinnere eingeleitet wird,
dadurch **gekennzeichnet**, daß an bzw. in dem weiteren Kanal eine Heizeinrichtung für das Gas vorgesehen ist, die das Gas auf Körpertemperatur, d.h. in etwa auf 37°C erwärmt.

2. Instrument nach Anspruch 1,
dadurch **gekennzeichnet**, daß die Heizeinrichtung in das Instrument selbst integriert ist.

3. Instrument nach Anspruch 1,
dadurch **gekennzeichnet**, daß die Heizeinrichtung in einem separaten Gehäuse angeordnet ist, das einen Fluideinlaß und einen Fluidauslaß aufweist, der über ein kurzes Schlauchstück mit dem proximalen Fluideinlaß des eigentlichen Instruments verbunden ist.

4. Instrument nach einem der Ansprüche 1 bis 3,
dadurch **gekennzeichnet**, daß die Heizeinrichtung wenigstens eine elektrische Heizwendel aufweist.

5. Instrument nach Anspruch 4,
dadurch **gekennzeichnet**, daß die Heizwendel den weiteren Kanal, durch den das Fluid geleitet wird, umgibt.

6. Instrument nach Anspruch 4 oder 5,
dadurch **gekennzeichnet**, daß die Heizwendel quaderförmig aufgebaut ist.

7. Instrument nach Anspruch 4 oder 5,
dadurch **gekennzeichnet**, daß die Heizwendel flach ausgebildet ist.

8. Instrument nach einem der Ansprüche 1 bis 2 oder 4 bis 7,
dadurch **gekennzeichnet**, daß die Heizeinrichtung in dem Teil des Instruments vorgesehen ist, der außerhalb des menschlichen Körpers verbleibt.

9. Instrument nach einem der Ansprüche 1 bis 2 oder 4 bis 7,
dadurch **gekennzeichnet**, daß die Heizeinrichtung den Teil des Instruments umgibt, der das optische System des Systems und insbesondere eines Endoskops aufnimmt.

10. Instrument nach Anspruch 9,
dadurch **gekennzeichnet**, daß die Heizeinrichtung in einem separaten Gehäuse vorgesehen ist, das einstückig mit dem in herkömmlicher Weise ausgebildeten Instrument verbunden ist.

11. Instrument nach einem der Ansprüche 1 bis 10,
dadurch **gekennzeichnet**, daß die Heizeinrichtung wenigstens einen Temperaturfühler aufweist, dessen Ausgangssignal an eine Regeleinrichtung angelegt ist, die die Leistung regelt, mit der die Heizeinrichtung beaufschlagt wird.

12. Instrument nach Anspruch 11,
dadurch **gekennzeichnet**, daß mindestens zwei Temperaturfühler vorgesehen sind, deren Ausgangssignale zur Überprüfung der Funktion der Temperaturfühler verglichen werden.

13. Instrument nach Anspruch 11 oder 12,
dadurch **gekennzeichnet**, daß der oder die Temperaturfühler möglichst nahe am Kanal angeordnet sind, durch den das Fluid strömt.

14. Instrument nach einem der Ansprüche 1 bis 12,
dadurch **gekennzeichnet**, daß ein Temperaturfühler, der eine Sicherheitsabschaltung auslösen kann, möglichst nahe dem distalen Ende des Instruments angeordnet ist.

15. Instrument nach einem der Ansprüche 1 bis 14,
dadurch **gekennzeichnet**, daß eine Steuereinheit, an der gegebenenfalls das Ausgangssignal der Temperaturfühler anliegt, die Heizleistung steuert, mit der die Heizeinrichtung beaufschlagt wird.

16. Instrument nach einem der Ansprüche 1 bis 15,
dadurch **gekennzeichnet**, daß das Instrument ein an sich bekannter Trokar ist.

17. Instrument nach einem der Ansprüche 1 bis 16,
dadurch **gekennzeichnet**, daß das Instrument ein an sich bekanntes Laparoskop für operative Anwendungen ist.

## Claims

1. Endoscopic instrument comprising
- a section remaining outside the human body, on which a gas connector (3; 4') is provided that serves for connection of a connecting tube that connects the instrument with an insufflation device conveying gas such as CO₂ at flow rates suitable for insufflation of body cavities, particularly at rates up to 40 l/min, and
- an elongate section adapted to be introduced into the human body, which comprises at least one duct (2) extending along the longitudinal axis of this section, which duct is connected via a further passage to said gas connector (3, 4') such that gas will be introduced into the interior of the body,
**characterised** in that a means is provided on or in said further passage for heating the gas, which heats the gas to body temperature, i.e. to 37 °C approximately.

2. Instrument according to Claim 1,
**characterised** in that said heating means is integrated into the instrument as such.

3. Instrument according to Claim 1,
**characterised** in that said heating means is disposed in a separate housing comprising a fluid inlet and a fluid outlet that is connected to the proximal fluid inlet of the instrument proper via a short tube length.

4. Instrument according to any of the Claims 1 to 3,
**characterised** in that said heating means comprises at least one electrical heating spiral.

5. Instrument according to Claim 4,
**characterised** in that said heating spiral surrounds said further passage through which the fluid is passed.

6. Instrument according to Claim 4 or 5,
**characterised** in that said heating spiral has a square structure.

7. Instrument according to Claim 4 or 5,
**characterised** in that said heating spiral has a flat configuration.

8. Instrument according to any of the Claims 1 to 2 or 4 to 7,
**characterised** in that said heating means is provided in that part of the instrument which remains outside the human body.

9. Instrument according to any of the Claims 1 to 2 or 4 to 7,
**characterised** in that said heating means surrounds that part of the instrument which accommodates the optical system of the overall system and of an endoscope in particular.

10. Instrument according to Claim 9,
**characterised** in that said heating means is provided in a separate housing that is integrally connected to the instrument which is configured in a conventional design.

11. Instrument according to any of the Claims 1 to 10,
**characterised** in that said heating means comprises at least one thermometric sensor whose output signal is applied to a feed-back control means that controls the power applied to said heating means.

12. Instrument according to Claim 11,
**characterised** in that at least two thermometric sensors are provided whose output signals are compared for checking the function of said thermometric sensors.

13. Instrument according to Claim 11 or 12,
bin that said thermometric sensor or sensors is/are disposed as close as possible to the passage through which the fluid passes.

14. Instrument according to any of the Claims 1 to 12,
**characterised** in that a thermometric sensor capable of triggering a safety cut-out means is disposed as close as possible to the distal end of the instrument.

15. Instrument according to any of the Claims 1 to 14,
**characterised** in that a controller, to which possibly the output signal of said thermometric sensors is applied, controls the heating energy applied to said heating means.

16. Instrument according to any of the Claims 1 to 15,
**characterised** in that the instrument is a trocar known per se.

17. Instrument according to any of the Claims 1 to 16,
**characterised** in that the instrument is a laparoscope known per se for applications in surgery.

## Revendications

1. Instrument endoscopique comprenant
- une partie qui reste à l'extérieur du corps humain, sur lequel un raccord de gaz (3; 4') est disposé, qui sert à relier un tuyau de connexion, qui relie l'instrument à un appareil d'insufflation, lequel passe du gaz comme du CO₂ aux débits qui sont appropriés à insuffler des cavités dans le corps, en particulier aux débits jusqu'à 40 l/min, et
- une partie allongée, qui est apte à être introduite dans le corps humain, qui comprend au moins un passage (2), qui s'étend le long de l'axe longitudinal de cette partie, ce passage étant relié moyennant un autre passage audit raccord de gaz (3, 4") d'une manière que du gaz soit introduit dans l'intérieur du corps,
**caractérisé** en ce qu'un moyen de chauffage est disposé sur ou dans ledit autre passage pour chauffer le gaz, qui chauffe le gaz à la température du corps, c'est-à-dire à 37 °C environ.

2. Instrument selon la revendication 1,
**caractérisé** en ce que ledit moyen de chauffage est intégré dans l'instrument directement.

3. Instrument selon la revendication 1,
**caractérisé** en ce que ledit moyen de chauffage est disposé dans un carter séparé, qui comprend un orifice d'admission de fluide et un orifice d'évacuation de fluide, qui est reliée à l'orifice d'admission de fluide proximale de l'instrument en soi via un court tronçon de tuyau.

4. Instrument selon une quelconque des revendications 1 à 3,
**caractérisé** en ce que ledit moyen de chauffage comprend au moins une spirale du filament chaud électrique.

5. Instrument selon la revendication 4,
**caractérisé** en ce que ladite spirale du filament chaud entoure ledit passage additionnel, à travers duquel s'écoule le fluide.

6. Instrument selon la revendication 4 ou 5,
**caractérisé** en ce que ladite spirale du filament chaud a une structure parallélépipède.

7. Instrument selon la revendication 4 ou 5,
**caractérisé** en ce que ladite spirale du filament chaud a une forme plate.

8. Instrument selon une quelconque des revendications 1 à 2 ou 4 à 7,
**caractérisé** en ce que ledit moyen de chauffage est placé dans cette partie de l'instrument, qui reste à l'extérieur du corps humain.

9. Instrument selon une quelconque des revendications 1 à 2 ou 4 à 7,
**caractérisé** en ce que ledit moyen de chauffage entoure cette partie de l'instrument, qui reçoit le système optique de l'appareil et d'un endoscope en particulier.

10. Instrument selon la revendication 9,
**caractérisé** en ce que ledit moyen de chauffage est disposé dans un carter séparé, qui est intégralement relié à l'instrument, dont la configuration est conventionnelle.

11. Instrument selon une quelconque des revendications 1 à 10,
**caractérisé** en ce que ledit moyen de chauffage comprend au moins un palpeur de température dont le signal de sortie est appliqué à une unité de réglage, qui commande l'énergie appliquée audit moyen de chauffage.

12. Instrument selon la revendication 11,
**caractérisé** en ce qu'au moins deux palpeurs de température sont disposés, dont les signaux de sortie sont comparés afin de surveiller la fonction desdits palpeurs de température.

13. Instrument selon la revendication 11 ou 12,
**caractérisé** en ce que ledit ou lesdits palpeur(s) de température est/sont disposé(s) aussi près du passage que possible, à travers duquel s'écoule le fluide.

14. Instrument selon une quelconque des revendications 1 à 12,
**caractérisé** en ce qu'un palpeur de température, capable de déclencher un moyen interrupteur de sécurité, est disposé aussi près de l'extrémité distale de l'instrument que possible.

15. Instrument selon une quelconque des revendications 1 à 14,
**caractérisé** en ce qu'une unité de commande, auquel le signal de sortie desdits palpeurs de température est éventuellement appliqué, commande l'énergie de chauffage, qui est appliquée audit moyen de chauffage.

16. Instrument selon une quelconque des revendications 1 à 15,
**caractérisé** en ce que l'instrument est un trocart connu en soi.

17. Instrument selon une quelconque des revendications 1 à 16,
**caractérisé** en ce que l'instrument est un laparoscope connu en soi pour des applications en chirurgie.
